(19) Europäisches Patentamt
European Patent Office
Office européen des brevets

(11) Publication number: 0 249 263 A2

# (12) EUROPEAN PATENT APPLICATION

(21) Application number: 87200904.8

(51) Int. Cl.[4]: C08G 59/00 , C08G 59/02

(22) Date of filing: 14.05.87

(30) Priority: 09.06.86 US 871950

(43) Date of publication of application: 16.12.87 Bulletin 87/51

(84) Designated Contracting States: DE FR GB IT NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Dewhirst, Kenneth Carl
410 Bayou Cove
Houston Texas 77042(US)

(74) Representative: Aalbers, Onno et al
P.O. Box 302
NL-2501 CH The Hague(NL)

## (54) Thermoplastic polymer composition having thermosetting processing characteristics.

(57) The present invention relates to a new thermoplastic polymer composition having the processing characteristics of a thermosetting polymer along with an improved balance of properties including improved modulus/glass transition temperature/toughness balance. These new polymer compositions are prepared by reacting certain amine compounds with certain diepoxide compounds to form linear units which are lightly crosslinked. Also disclosed and claimed are processes for preparing such compositions, cured compositions and end-use applications.

EP 0 249 263 A2

Xerox Copy Centre

# THERMOPLASTIC POLYMER COMPOSITION HAVING THERMOSETTING PROCESSING CHARACTERISTICS

The present invention relates to novel thermoplastic polymer compositions which may be converted into thermoset composite structures by typical thermosetting resin methods. In particular, the present invention relates to thermoplastic compositions having flexible portions and bulky stiff portions in their molecular structures, prepared by reacting a polyepoxide with a polyphenol to form linear molecules and crosslinking to a certain degree the resulting molecules.

Epoxy compositions and their curing techniques are well known, and the patents issued on curable epoxy compositions number in the thousands. It will be appreciated that each and every one of the known epoxy-curing systems has its pros and cons and that there remains a continuing need to develop better epoxy compositions.

There is, in particular, an increasing need in the aerospace and automotive sectors for high performance thermosetting compositions or matrices for fibre reinforced composites. Fibre reinforced composites are very desirable in aerospace applications because they can offer a combination of good stiffness, strength and are light weight. Increasingly, the aerospace manufactures have demanded higher performance from the thermoset resins used in fibre reinforced composites. These higher performance thermoset resins are expected to possess these following characteristics:

-good mechanical properties at temperatures above about 90 °C
-good thermal oxidative stability
-good toughness properties, including good impact resistance
-good fatigue properties
-good chemical and solvent resistance
-good fire resistance
-high resistance to humidity, e.g., the "hot-wet" properties of the composite must remain high.

A further, and very important property of such systems is that the composite must have acceptable processing characteristics. For example, the current techniques for manufacturing aerospace components typically involves the use of prepregs and laminates, which are cured by applying heat and pressure in a vacuum bag/autoclave-type apparatus. Therefore, the most desirable thermosetting resin compositions should be processable on the standard equipment currently utilized in the aerospace industry.

A broad spectrum of thermosetting epoxy resin systems is currently being used by the aerospace industry, primarily as composite matrices and adhesives. As a class, epoxy resin systems are very versatile materials offering, as mentioned above, chemical resistance, high adhesive strength, good electric properties and are easy to use or process into composites. However, to improve their high temperature properties, such current epoxy resin systems must be highly crosslinked. This crosslinking, however, results in generally lower toughness.

There are currently various engineering thermoplastics (not thermosetting polymers) that offer excellent high temperature properties along with high toughness. One such example currently being investigated is the use of poly(ether-ether)ketone ("PEEK") as the matrix. However, there are processing problems with the use of PEEK and other similar engineering thermoplastic resins since such materials not only are difficult to process on thermoplastic apparatus (i.e., difficult to extrude), but also do not lend themselves to processing by the thermosetting techniques now currently in use by the aerospace industry.

What is needed is a resin system that not only combines the good property advantages of such high performance engineering thermoplastics such as PEEK, but is also processable as a thermosetting resin matrix.

Thermoplastic polyethers having relatively high impact strength are disclosed and claimed in US-A 3,637,590, while the process for preparing such polymers is claimed in US-A 3,306,872. Even though such polymers, which are based on the reaction product of certain diepoxides with certain bisphenols, have improved impact strength, such polymers lack adequate high temperature properties and solvent resistance for high performance applications. Similar compositions having improved impact strength are also disclosed in DE-A 2,142,579 where certain diepoxides are reacted with certain diphenols (e.g. 2,2-bis(4-hydroxynaphth-1-yl)propane) to produce polymers for eyeglasses.

Other epoxy systems such as those disclosed in US-A 4,473,674, are touted for aerospace applications. The composites described in this reference are based on multifunctional epoxies, such as tetraglycidylmethylenedianiline. Such systems also have important deficiencies as discussed in the various examples which follow.

The present invention aims at achieving a thermoplastic composition having the processing characteristics of a thermosetting polymer along with an improved balance of properties including solvent resistance and improved modulus/glass transition temperature/toughness balance. Thereto the invention relates to a composition comprising linear molecules having the repeating structures prior to crosslinking of the general formula:

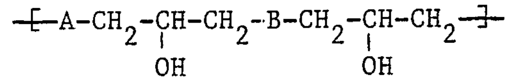

where A is selected from the group consisting of:

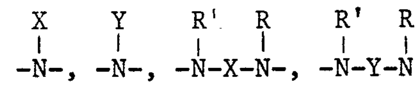

and mixtures thereof, and B is selected from the group consisting of:

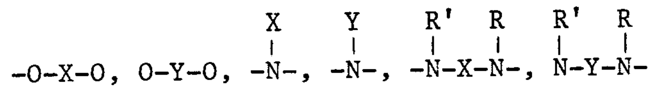

where R and R′ are selected from the group consisting of unsubstituted or inertly substituted $C_1$-$C_{20}$ aliphatic, cycloaliphatic or aromatic hydrocarbyl groups, and wherein said repeating structures are lightly crosslinked such that between 1 and 50 of said repeating structures per 100 total repeating structures are crosslinked to repeating structures of other molecules, and where:

a) "X" represents a stiff segment comprising stiff units (SU) and optional flexible units (FU), which stiff units and flexible units are interconnected;

b) "Y" represents a flexible segment comprising stiff units (SU′) and optional flexible units (FU′) which stiff units and flexible units are interconnected;

c) said stiff units, SU and SU′, are independently selected from the group consisting of substituted and non-substituted aromatic rings, cycloaliphatic rings and non-interfering heterocyclic rings;

d) said flexible units, FU and FU′, are independently selected from the group consisting of

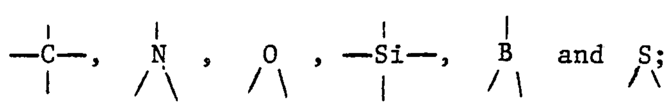
and

e) the number of stiff segments in the resulting polymer compositions is "a", the number of flexible segments in the resulting polymer compositions is "b", and the ratio of $\frac{a}{a+b}$ is less than or equal to one; and

f) the ratio of the number of stiff units to flexible units in said stiff segment (SU/FU) is equal to or greater than the ratio of the number of stiff units to flexible units in said flexible segment (SU′/FU′).

As shown in the examples which follow, Applicants have discovered a new method for preparing novel polymers wherein it is now possible to obtain both high temperature performance and high toughness, i.e., Applicants have discovered a means to uncouple the usual temperature/toughness balance relationship. In particular, in a preferred embodiment Applicants have prepared polymers having the following property set:

Glass transition temperature, Tg = 168 °C (DSC)

Fracture toughness, $K_q$ = 2.5 $KSI.in.^{0.5}$ (Compact Tension)

Flex modulus, E = 520 KSI (Dry R.T.)
490 KSI (Wet 200 °F)

Water gain, $\Delta W/W_o$ = 0.8% (saturation).

There are two basic aspects to the present invention: one involves the process for making certain thermoplastic polymers and the other involves the polymers as compositions-of-matter.

## I. Process

In a broad sense, the present invention relates, as a minimum, to the mixture of an amine component and a diepoxide component to make a prepolymer composition, which may be stored for later reaction or which may be reacted with a condensation catalyst.

### A. Amine Component

The amine component employed in making the polymers of the present invention is selected from the group consisting of primary amines, bis secondary amines and mixtures thereof.

The primary amines will have the general formula

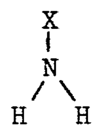
or
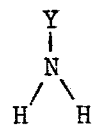

or a mixture thereof where "X" and "Y" are the stiff segments or flexible segments referred to before.

The bis secondary amines will have the general formula:

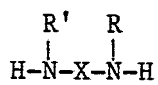
or
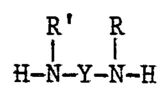

or a mixture thereof where "X" and "Y" are the stiff segments or flexible segments referred to before and R and R′ are unsubstituted or inertly-substituted $C_1$-$C_{20}$ aliphatic, cycloaliphatic or aromatic hydrocarbyl groups. Preferably R and R′ are $C_1$-$C_{10}$ alkyl groups. Examples of R and R′ include methyl, ethyl, isopropyl, cyclohexyl, benzyl, tolyl and the like.

Examples of primary monoamines include aniline (phenylamine), 2,6-dimethylaniline, 2,4-dimethylaniline, 2,6-diethylaniline, N-aminophthalimide, 2,6-diisopropylaniline, tolylamine, α-naphthylamine, 3-aminobenzothiophene, 1-aminoadamantane, and norbornylamine. Preferred primary monoamines include aniline, 2,6-dimethylaniline and 2,6-diethylaniline with 2,6-diethylaniline being most preferred.

Examples of bis secondary amines include
N,N′-dimethyl-p-phenylenediamine,
N,N′-dicyclohexyl-p-phenylenediamine,
bis-(N-sec-butyl-4-aminophenyl)-methane,
αα′-bis(N-sec-butyl-4-aminophenyl)-p-diisopropylbenzene,
9,9-bis-(N-methyl-4-aminophenyl)fluorene,
N,N′-dimethyl-4,4′-diaminodiphenyl sulphone, and
α,α′-bis(1-hydroxy-2-naphthyl)-para-diisopropylbenzene.

An important aspect of the present invention is the selection of stiff units and flexible units such that the resulting polymer molecules have the appropriate type and ratio of stiff units to flexible units.

By use of the term "flexible units " are meant those units that permit rotation at an angle. Examples of such flexible units are

| Broad group | Examples | | | |
|---|---|---|---|---|
| 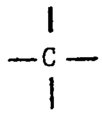 | 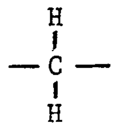 | 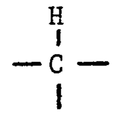 | 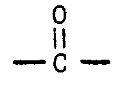 | 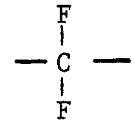 |
| 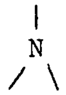 | 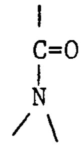 | | | |
| 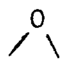 | 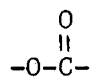 | | | |
| 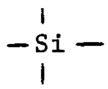 | 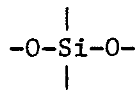 | | | |
| 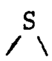 | 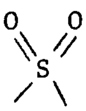 | | | |

The stiff units are selected from the group consisting of substituted or non-substituted aromatic rings, cycloaliphatic rings and heterocyclic rings. The aromatic rings are inertly substituted or un-substituted benzene radicals. Substituted benzene radicals have substituents which do not interfere in the process independently selected from the group consisting of Cl, Br or $C_1$-$C_5$ alkyl groups. Annulation of benzene rings gives rise to

naphthalene 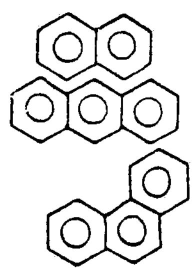 (SU=2),

anthracene (SU=3) and

phenanthrene (SU=3),

and the like.

The cycloaliphatic rings are substituted or un-substituted $C_5$ or $C_6$ hydrocarbon radicals. Substituted cycloaliphatic rings are analogous to substituted aromatic rings. Un-substituted rings include, by way of example, cyclopentane, cyclohexane and cyclohexene. Annulation of cycloaliphatic rings gives rise to

decalin 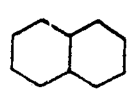 (SU=2)

[2.2.2]bicycloctane (SU=3)

norborane (SU=3)

adamantane ($C_{10}H_{16}$) 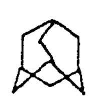 (SU=4)

and the like.

The term heterocyclic rings refers to substituted or un-substituted 5-6 membered heterocyclic radicals. Examples of 5-6 membered heterocyclic radicals are radicals of pyrrole, pyridine, furan, thiophene, imidazole, oxazole, thiazole, dibenzothiophene and carbazole.

Regarding the selection of heterocyclic structures, O and S heterocycles are generally suitable. In the case of N derivatives, however, care must be exercised such that the N is not strongly basic so that homopolymerization of the epoxide occurs. For example,

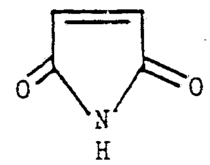

is suitable but

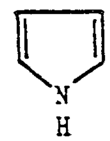

may not be suitable by itself (however the carbazole analogue is suitable since the N is not strongly basic there).

B. Diphenoxy Component

A portion, e.g. about one mole percent to about 99 mole percent. of said amine component can be replaced with a diphenoxy component. Preferably, when this replacement is desired, about 25 to about 75 mole percent of said amine component is replaced with said diphenoxy component.

In a preferred embodiment the diphenoxy components employed herein have the structure HO-X-OH or HO-Y-OH where "X" represents the stiff segment specified above and "Y" represents the flexible segment specified above.

One group of diphenoxy components particularly useful herein are those mentioned in US-A 3,546,145. Specifically, those useful components are those phenoxy compounds of the formula

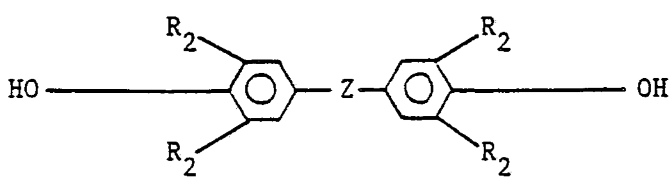

where each $R_2$ substituent is independently selected from H, Cl, Br or $C_1$-$C_5$ alkyl and Z is a substituent having flexible units (FU or FU') and stiff units (SU or SU') where Z represents a gem-bivalent radical having 1 or 2 aromatic hydrocarbon rings and a gem-bivalent non-aromatic ring selected from the group consisting of a ring of 5 carbon atoms, a ring of 6 carbon atoms one of which carbon atoms may bear an oxo oxygen atom, and a ring of 5 carbon atoms and one oxygen atom, said gem-bivalent non-aromatic ring being fused to said aromatic hydrocarbon rings. Particularly useful are those components where Z is

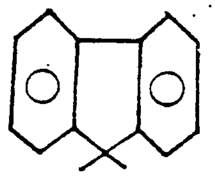

or

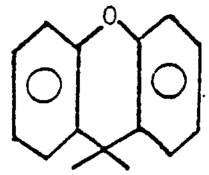

Specific examples include the following bisphenols:
9,9-bis(4-hydroxyphenyl)fluorene,
1,1-bis(4-hydroxyphenyl)-indane,
9,9-bis(4-hydroxyphenyl)xanthene,
10,10-bis(4-hydroxyphenyl)anthrone,
9,9-bis(4-hydroxyphenyl)phenanthrone.
Other useful bisphenols include phenolphthalene,
9,9-bis(4-hydroxyphenyl)-9,10-dihydroanthracene
, 9,9-bis(4-hydroxyphenyl)-10,10-diphenyl-9,10-dihydroanthracene,
3,3-bis(4-hydroxyphenyl)-4,5-benzodihydrofuran, and the like.

Another group of diphenoxy components useful herein are the imides represented by the formula

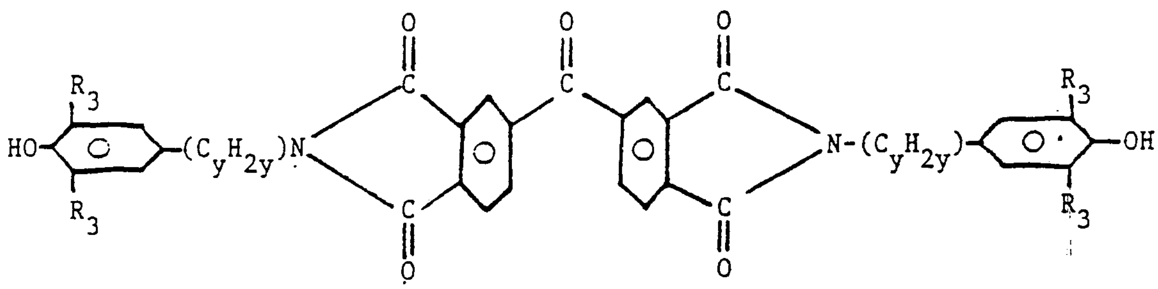

wherein each of $R_3$ is the same or different (lower) alkyl group of from one to four carbon atoms; and y has a value of from 0 to 3. Such diphenoxy compounds are disclosed in US-A 3,821,162.

Still another group of diphenoxy compounds are those based on phthalocyanine. Such compounds include $PcSi(R_5)_2$ in which $R_5$ = OH, or

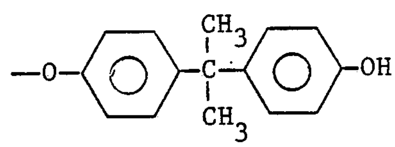

and Pc stands for a phthalocanine ring structure.

Still another group of diphenoxy compounds are those shown below. Additional aromatic, cycloaliphatic or heterocyclic rings may be annulated as desired:

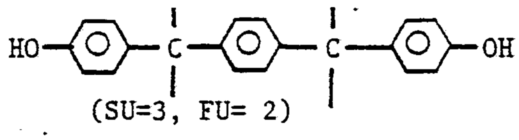

(SU=3, FU= 2)

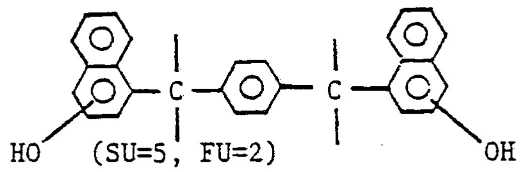

(SU=5, FU=2)

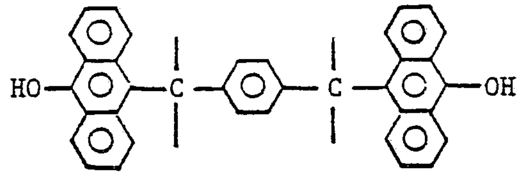

(SU=7, FU=2)

This particular group of diphenoxy compounds are distinguished from diphenoxy compounds such as BPA and the like, by the presence of 2 or more flexible units C .

If desired, the diphenoxy compounds described above may be substituted in part (or even in whole in certain cases) with other diphenols, represented by the general formula

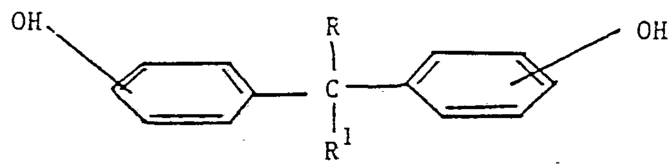

In which R and $R^1$ when taken collectively with the connector carbon C are selected from the group consisting of cyclohexyl and alkyl-substituted cyclohexyl, and when taken separately are from the group consisting of hydrogen, alkyl, cyclohexyl, phenyl, alkyl-substituted cyclohexyl, alkyl substituted phenyl, halogen substituted cyclohexyl and halogen substituted phenyl groups with the total number of carbon atoms in the group or groups attached to said connector carbon atom not exceeding eighteen and the number of carbon atoms in any of said alkyl substituent groups not exceeding six. The preferred phenols have the hydroxyl groups in the 4,4′ positions, but compounds with hydroxyls in the 2,2′, 3,3′, 2,4′ and other arrangements may also be used. R and $R^1$ suitable are methyl, ethyl, isobutyl, n-nonyl, n-heptadecyl and the like. Other dihydric phenols may also be employed, excepting those which have two hydroxyl groups in ortho positions on a single benzene ring.

C Diepoxide Component

The second reactant in the condensation process, the diep oxide, is a compound having two vicinal epoxide groups (oxirane rings) in terminal (or optionally non-terminal) positions in the molecule, usually in the form of an oxygen atom bound to two terminal carbons of an alkyl group, though the epoxide may also be on a ring, such as a cyclohexyl ring. Suitable diepoxides are terminal diepoxyalkanes, e.g., 1,2-epoxy-3,4-epoxybutane, 1,2-epoxy-5,6-epoxyhexane, 1,2-epoxy-7,8-epoxyoctane and the like. Others are terminal diepoxides containing ether linkages, such as bis(2,3-epoxypropyl)ether and bis(2,3-epoxy-2-methylpropyl)-ether; diglycidyl ethers of alpha, omega glycols such as the diglycidyl ethers of ethylene glycol, trimethylene glycol, and tetramethylene glycol; and diglycidyl ethers of dihydric phenols.

Diglycidyl ethers of the dihydric phenols referred to above are generally suitable for use in this invention. One may suitably use the diglycidyl ether of the same phenol which is employed as the other reactant. Thus, for example, bisphenol fluorenone is suitably condensed with diglycidyl ether of bisphenol fluorenone. Useful resins can also be prepared by condensing a dihydric phenol with the diglycidyl ether of a different dihydric phenol. For example, useful condensation products have been prepared according to this invention from the diglycidyl ether of BPA and the dihydric phenol prepared from phenol and fluorenone.

In preparing the products of this invention the epoxy reagent may be a pure diepoxide or a crude mixture containing a substantial proportion of diepoxide, e.g., 70% or more. It is important, however, that the crude reagent is free of monoepoxide and of monohydric alcohol or phenol. The polyepoxides used herein can have the structure

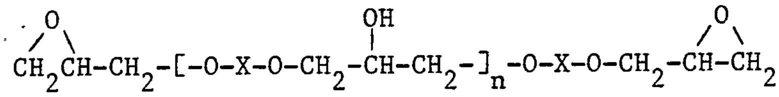

or

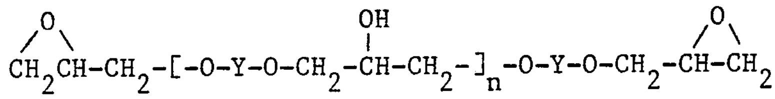

The number "n" has a value of from 0 to 6, preferably from 0 to 2, most preferably zero.

A particularly preferred diepoxide is the diglycidyl ether of BPA. Such diepoxides are available from Shell Chemical Company as EPON Resins 825 and 828. Shell EPON Resins 825 and 828 are diepoxides of BPA.

D. Selection of Stiff Units and Flexible Units for Stiff Segments and Flexible Segments

A key aspect of the present invention is the selection and location of the stiff units (SU and SU') and flexible units (FU and FU') for the stiff segments (X) and flexible segments (Y). As discussed above, the stiff segment (X) may be located in either the diphenoxy component or diepoxide component or in both components. For ease of synthesis it is preferred that the stiff segment be in either the diepoxide component or the diphenoxy component. The selection will depend upon the particular components to be employed. For example, since the diepoxide of BPA is readily available, it may be preferable to use such a resin in the synthesis. Since the diepoxide of BPA has one flexible unit and two stiff units, it has an

$$\frac{FU'}{SU'} \text{ of } \frac{2}{1}$$

or 2 and will therefore be the flexible segment Y. Then one must use a diphenoxy component having sufficient number of stiff units (SU) and flexible units (FU) to obtain the desired ratios and polymer characteristics.

More particulars on these ranges and selections are found in the discussion of the Structures of the Resulting Polymers.

E. Catalyst and Reaction Conditions

The condensation reaction between the diphenoxy component and the diepoxide component requires the presence of a condensation catalyst, typically a basic condensation catalyst. The catalyst, may, for example, be added as a concentrated aqueous solution of sodium or potassium hydroxide or a sodium salt of a phenol. One may also use halides, carboxylates or other nucleophiles. It is sometimes desirable to use as catalyst a sodium salt of the same dihydric phenol which is used as a reactant. These salts are generally solids which are dissolved in the reaction mixture. It has been found that very satisfactory results are also obtained when using concentrated aqueous sodium hydroxide or benzyltrimethyl ammonium hydroxide. When the catalyst is added as an aqueous solution, a concentrated solution is used since it is not desirable to have more than a small amount of water present in the reaction mixture.

The concentration of catalyst present during the condensation reaction must be held to a low value; otherwise branched polymers of low impact value are produced. However, it has also been found that reaction rates increase proportionately with catalyst concentration. The useful range of catalyst concentration is from 0.0001 to 0.100 mole per mole of the contained bisphenol. For best results the concentration is preferably between 0.001 and 0.010 mole per mole. It may occur that some of the catalyst reacts with

impurities in the reactants. This results in a reduction of the rate of reaction and can stop the reaction prematurely. Adding a further amount of catalyst then permits the reaction to continue. It has been found that basic catalyst reacts with saponifiable chlorine if the latter is present in the diglycidyl ether reactant. It is therefore useful to add initially an extra amount of catalyst, sufficient to react with such chlorine, to prevent slowing down of the reaction.

It is preferred to keep the water content of the reaction mixture as low as possible, preferably below 0.5% by weight, more preferably below 0.2% by weight, and still more preferably below 0.12% by weight. In any event, the water content is to be maintained so as not to exceed about 1 percent by weight.

Careful control of the ratio of dihydric phenol and diglycidyl ether in the reaction mixture is of greatest importance in order to obtain a product having the desired characteristics. When technical grades of one or several reagents are employed, the correct ratio is maintained by determining the epoxy equivalence and the amine hydrogen equivalency of the reagents and carrying out the reaction with a mixture which contains not less than 0.8 amine hydrogen per epoxide group and not more than 1.2 amine hydrogen per epoxide groups.

Best results are obtained with phenolic hydroxide/epoxy ratios in the range from 0.96 to 1.04.

The reaction is typically carried out in solution in a solvent which meets the following criteria: (1) It is capable of maintaining reactants and reaction products in solution, at reaction temperatures, in the concentrations employed. These concentrations generally range between 20 and 60 percent by weight of the total reaction mixture. When the original concentration is high, it is generally necessary to add additional solvent during the course of the reaction to reduce the viscosity of the mixture and to maintain the product in solution. (2) It does not react significantly with epoxide groups or phenolic hydroxyl groups. Water and alcohols, for example, tend to interreact with the reactants and are therefore not suitable as solvents. (3) It is readily and completely removable from the final reaction mixture to permit recovery of a resin substantially completely free of solvent. Desired high impact resistance is a property which requires complete removal of solvent. In the production of resin for use in moulding, extrusion, and the like, solvent is removed from the reaction mixture. In the production of resin for surface coatings, the resin may remain associated with solvent until it is actually applied as a coating and the solvent is removed by evaporation under suitable conditions. (4) Its boiling point must be such that the reaction can be carried out at 75 °C to 150 °C at a practical pressure. The solvent may be a mixture of individual compounds.

Useful solvents which meet those criteria are, for example, certain ketones, halogenated hydrocarbons and ethers. Methyl ethyl ketone is a preferred solvent. Cyclohexanone, methyl isobutyl ketone and the other ketones may be used. Chloroform, 1,2-dichloroethane and other chlorinated hydrocarbons may be used, particularly in admixture with ketones. Ethers, such as dioxane, tetrahydrofuran, dimethoxyethane and lower alkyl (methyl or ethyl) ethers of ethylene glycol are suitable, alone or in admixture with ketones. Other solvents which meet the above criteria may be employed if desired, such as N-methyl pyrrolidone.

While in the examples which follow the synthesis was performed in solution, it is also possible (and desirable in some cases) to do the synthesis in the absence of solvent, i.e. as a melt. In such cases it may be desirable to use the diepoxide containing the stiff segment since the melting point of diepoxide component is usually much lower than the melting point of the corresponding diphenoxy component.

## II. Resulting Polymers

### A. Structures

As mentioned above, a key aspect of the present invention is the selection and location of the stiff units (SU and SU') and the flexible units (FU and FU') for the stiff segments (X) and flexible segments (Y). Great latitude is provided for the selection and location of the particular components. For the most part the properties and performance of the resulting polymers depend primarily on the relative number of stiff units and flexible units (each such unit being assigned a value of one). However, there are certain important ratios and values that need to be followed.

Note that it is not necessary that the segments contain flexible units. For example, BPFL does not contain flexible units and is perfectly satisfactory.

The first important ratio is the average number of total stiff units divided by the average number of total flexible units. For a preferred group of structures this ratio must satisfy the equation:

$$\frac{\left(\frac{a}{a+b}\right) SU + \left(\frac{b}{a+b}\right) SU'}{\left(\frac{a}{a+b}\right) FU + \left(\frac{b}{a+b}\right) FU'}$$

Preferably this ratio is from 4 to 20, most preferably from 5 to 10. This ratio is important because it is an important factor in determining the Tg, or heat resistance of the polymer.

The second important ratio is that SU/FU must be equal to or greater than SU'/FU', preferably greater. In other words, the stiff segment (X) must have an equal or higher ratio of SU/FU than the flexible segment (Y) ratio of SU'/FU'. This is important because it helps determine the $T_g$/toughness balance. Preferably SU/FU > SU'/FU' + 0.5. In a preferred embodiment SU'/FU' is between 1 and 4, preferably between 2 and 3. For example, where it is preferred to use the diglycidyl ether of BPA, the SU'/FU' ratio is 2/1 or 2.

The third important ratio is the relative amounts of stiff segments and flexible segments, i.e. $\frac{a}{a+b}$. In the broadest case the ratio of $\frac{a}{a+b}$ is more than zero and less than or equal to 1. The preferred ratios of $\frac{a}{a+b}$ are between 0.2 and 0.8, more preferably between 0.3 and 0.7, most preferably between 0.4 and 0.6. It is shown in the examples which follow that a most preferred ratio of $\frac{a}{a+b}$ is between 0.4 and 0.6 with optimum overall properties (espe-cially solvent resistance) occurring when the ratio of $\frac{a}{a+b}$ is equal to 0.5.

## B. Light Crosslinking

Another important aspect of the present invention relates to the light crosslinking of the thermoplastic polymer molecules to form the resulting polymer matrix. The concept and process for light crosslinking of such polymers is another novel and unobvious aspect of the present invention. In the broadest sense, light crosslinking refers to the crosslinking of between 1 and 50 out of each 100 repeat units to repeat units of other molecules, e.g. formulas I or II of said thermoplastic polymer. Preferably, the crosslinking density is between 2 and 40 out of 100, more preferably between 5 and 25 repeat units per 100 repeat units.

"Light crosslinking" is distinguished from the normal crosslinking or curing of epoxy resins where the crosslink density approaches 100 (stoichiometric) molecules or repeat units per 100 molecules or repeat units.

There are basically three different techniques that may be used to obtain lightly crosslinked matrices. One technique involves the use of a slightly greater number of diepoxide groups than phenolic groups (see earlier section on I.D. Catalyst and Reaction Conditions). When using this technique the repeat units will crosslink through the reaction of the secondary hydroxyl groups with the remaining epoxide groups. Once the thermoplastic polymer is prepared, it may be used alone or with a reinforcing fibre in an FRC-type (fibre reinforced composite) composition, wherein the polymer mass is heated to an elevated temperature (e.g. above 170 °C) and held at that temperature for the necessary time (typically about 2 to about 24 hours) to obtain crosslinking.

Another technique to obtain light crosslinking is to incorporate an appropriate amount of tri-or higher functional epoxide or tri-or higher functional phenolic or amine in the preparation of the thermoplastic polymer. The crosslinking agent, when added as a separate component, replaces a portion of the phenolic component or the epoxide component, as desired. For example, if 20% crosslinking agent is used, then 20% of the phenolic component is replaced on an equivalent basis.

Examples of suitable multifunctional epoxide polymers include Epon Resin 1031 and Epon Resin DPS-164. Epon Resin DPS-164 has the general formula

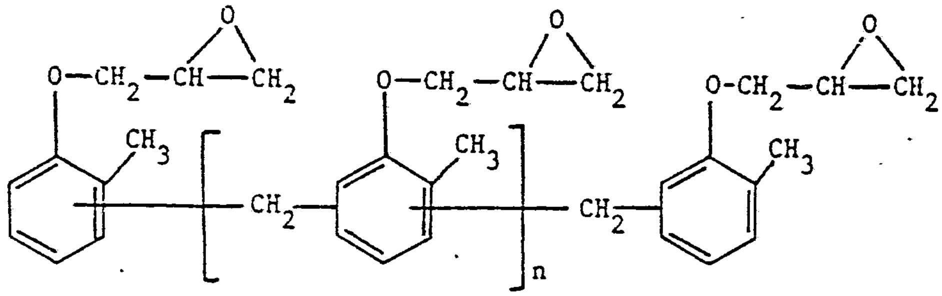

where n equals an average of 3.
Epon Resin 1031 has the structure

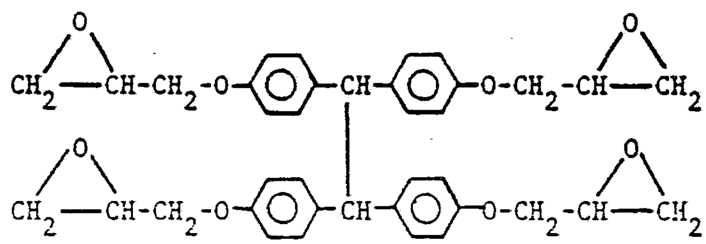

Other crosslinking agents include multifunctional amines such as EPON HPT™ Curing Agents 1061 and 1062, having the molecular structure:

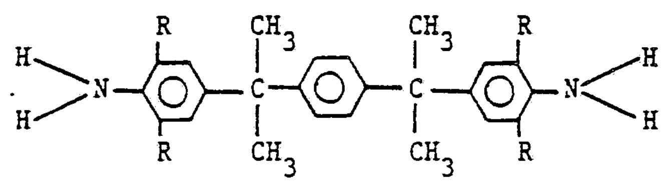

where R is H for CA 1061 and R is $CH_3$ for CA 1062.
Still other crosslinking agents include

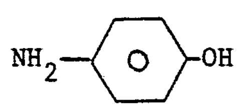 (PAP)

and

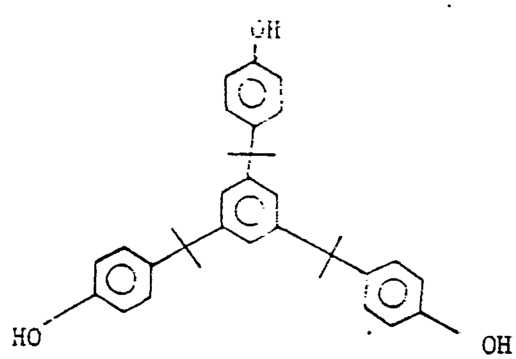

(TPA)

A third technique to obtain light crosslinking involves the addition of crosslinking agents, such as triepoxides, etc., to the resulting thermoplastic polymer. This technique is not preferred since it is more difficult to incorporate the crosslinking agent in the polymer after synthesis than before synthesis.

The amount of crosslinking agent chosen is selected to achieve the desired level of light crosslinking, as opposed to the normal crosslinking used for epoxy resins. Accordingly, when using a crosslinking agent such as EPON Resin 1031, the amount of equivalents used is 2 to 20%. Likewise, when the crosslinking agent is EPON HPT Curing Agent 1061, the amount of equivalents used is 5-50%.

## C. Formulations and Composites

The composition optionally, but preferably for high-performance applications such as automotive and aerospace, contains a reinforcing substrate. Suitable reinforcing materials include, for example, glass fibers, carbon fibres, Kevlar, boron, calcium carbonate, talc, alumina, asbestos and the like. The preferred fibrous reinforcing material for high-performance applications is selected from the group consisting of glass fibres, carbon fibres, boron fibres and Kevlar fibres, with continuous carbon fibre being most preferred. The fibrous reinforcing material will be present in the composition in an amount effective to impart increased strength to the cured composition, generally from about 40 to about 95 weight percent, usually from about 60 to about 80 weight percent, based on the weight of the total composition.

The present composition can be applied to the fibrous reinforcing material from the melt or solution by methods known in the art. Among the various processes useful with the present invention include resin transfer moulding (RTM), pultrusion, filament winding and the use of prepregs. Such methods are known in the art.

One method of current preferred interest involves the use of prepregs. In that system, the polymer composition/curing agent - impregnated substrate, or "pre-preg", or a laminate prepared from a plurality of prepregs, is then cured. When the system is based on Epon® 825 resin and the bisphenol of fluorenone, the curing is typically accomplished at a temperature of about 150 to about 200 °C for about 1 to 16 hours under vacuum or under a presence of 1 to 20 bar to form the structural composite article.

## D. Uses

The compositions of the present invention have particular application in the aerospace industry where the high performance obtainable with the present invention is required. In particular, RIM may be used to prepare large parts, such as helicopter blades. Prepregs may be used to prepare parts such as wings and the like. Filament winding may be used to prepare an entire fuselage, while pultrusion may be used to prepare parts having a constant cross section.

The invention composition can optionally include additives for control or modification of various properties of the composition in its cured or uncured state, including cure rate accelerators or retardants, tackifiers and the like.

To illustrate the present invention, the following illustrative embodiments and comparative examples are given. It is to be understood, however, that the embodiments and examples are given for the purpose of illustration only and the invention is not to be regarded as limited to any of the specific materials or conditions used in the specific embodiments.

As used in the following examples, Epoxy Resin A is a liquid glycidyl polyether of 2,2-bis(4-hydroxyphenyl)propane having an epoxide equivalent weight of 170-174 and an average molecular weight of about 345.

Epoxy Resin B is a liquid glycidyl polyether of 2,2-bis(4-hydroxyphenyl)propane having an epoxide equivalent weight of 180-195 and an average molecular weight of about 380.

The compositions were tested according to the following test procedures:

Flexural properties of neat resins were evaluated according to ASTM D790 method using 1/8 in. thick specimens. Specimens were tested both in Dry (at Room Temperature and ~75% R.H.) and Hot/Wet (after immersion in boiling water for 48 hours, test at 95 °C, 5 min. equilibration time) conditions.

Fracture toughness, $K_q$ , was measured using mini-compact tension specimens (see W.B. Jones, et al Am. Chem. Soc., Div. Polym. Chem., Polym. Prepr., 22, 1981). All specimens were slotted to a Chevron shape and then precracked with a razor blade.

Tensile properties were measured according to ASTM D638 method.

Swelling in solvents was evaluated by measuring weight gain per unit of initial weight after immersion in solvent for a specified time at room temperature.

Illustrative Embodiment I

In this example, two of the components used in making the polymers of the present invention are prepared.

Synthesis of α,α'-bis(1-hydroxy-2-naphthyl)-para-diisopropylbenzne (BNDB)

The synthesis of BNDB was accomplished using 1-naphthol, p-Diol (α,α'-dihydroxy-para-diisopropylbenzene), 1,1,2,-trichloroethane, and concentrated hydrochloric acid in a molar ratio of 8:1:4:1.02. Thus, a mixture of 1-naphthol, p-Diol, and 1,1,2-trichloroethane was heated as a slurry (which dissolves) to 65-70 °C with agitation under nitrogen. Conc. HCl was added slowly, controlling the temperature of the reaction to below 82 °C. The reaction was allowed to proceed for 50 minutes after the addition of the HCl at 70-80 °C. DNDB (M.W. 446) precipitated out of reaction in 90+% yield. The BNDB melts at 245-255 °C.

Synthesis of α,α'-bis[+1-(2,3-epoxypropoxy)-2-naphthyl]+-para-diisopropylbenzene (DGBNDB)

The synthesis of DGBNDB was accomplished using BNDB, epichlorohydrin (ECH), isopropanol (IPA), and water in a molar ratio of 1:15:13.5:13 and staging the addition of 20 %wt sodium hydroxide in water. To a slurry of BNDB in ECH, IPA and water at 70 °C under nitrogen was added slowly 1 mole of NaOH as 20 %wt NaOH in water per 1 mole of BNDB and reacted until the reaction mass was a solution. The brine was separated from the organic layer and discarded. This process of slow NaOH addition and brine removal was repeated three times more at 0.8, 0.8 and 0.4 moles of NaOH per mole of BNDB. After the last brine removal the organic phase was water washed and then vacuum-evaporated until neat, molten resin was obtained. The resin was dissolved 20 %wt in methylisobutyl ketone (MIBK) and then heated to 90 °C under nitrogen. A solution of 5 %wt NaOH in water equal to 25 %wt of the total reaction mass was charged to the resin solution and the heterogeneous mass allowed to react at 90-95 °C for two hours. The brine was removed and the MIBK solution was washed with water until the wash water was the same pH as it started. The MIBK was distilled at atmospheric pressure until the resin started to precipitate. The hot MIBK slurry was cooled to room temperature with stirring and the resin collected on a filter and dried in a vacuum oven at 100 °C overnight. The resin melts at 160 °C (approx.) and chromatographs as essentially 1 peak of 96 %+ area by reverse phase HPLC. The WPE is 280-284 (theory is 279) and the Sap. Cl is < 0.01 %wt.

N,N'-Dimethyl-α,α'-bis(4-aminophenyl)-p-diisopropylbenzene (NMADB)

N-methylaniline (8 mol), p-Diol (a+,a+'-dihydroxy-p-diisopropylbenzene) (1 mol), and Super Filtrol No. 1 catalyst (4 %wt) were heated in a reactor equipped with a Dean-Stark Trap and Nitrogen to a reaction temperature of 180 °C (removing the N-methylaniline/water azeotrope of p-Diol dehydration) and held at that temperature for 1 hour. The reaction mass was then cooled to a comfortable temperature and the Super Filtrol was removed by filtering through a Buchner funnel with Celite filter-aid. N-methylaniline was removed by vacuum distillation and the crude product was poured with stirring into methanol (approximately 1:1 MeOH/prdct.) and allowed to cool. The product crystallized out in 95+% purity by amine N titration and HPLC. The melting point was 115-122 °C.

Illustrative Embodiment II

In Illustrative Embodiment II, a polymer according to the present invention was made starting with DGBNDB and NMADB (prepared as in Illustrative Embodiment I), and EPON HPT™ CA 1061 curing agent (described earlier).

All of the materials used were recrystallized and thoroughly dried in a vacuum oven at 1mm Hg vacuum before they were used. Component specifications were as follows:

| | |
|---|---|
| DGBNDB | WPE= 282-284 (theo. 279); Sap. Cl. < .01 %wt |
| NMADB | Amine N = 5.312 Meq./gm (theo. 5.376 meq./gm); M.P. = 115-122 °C |
| Epoxy HPT CA 1061 | M.P. = 160-161 °C |

Calculations and Stoichiometry:

Theroretical Amine Hydrogen equivalent values were used in calculating charges for NMADB (0.74 eq) and HPT CA 1061 (0.26 eq). Actual WPE was used in calculating the charge for DGBNDB (1 eq).

Procedure:

The polymer was made by melting the three components together at 190 °C in a vacuum erlenmyer flask, degassing at 1 mm Hg until bubbling ceased, pouring the molten pre-polymer into a mould (made of two glass plates treated with a releasing agent, preheated in a forced draft oven at 190 °C) and curing the polymer for 24 hours. At the end of the cure cycle, the polymer-in-mould was taken out of the oven and allowed to cool below its Tg and then the plates were popped loose. The following properties were obtained:

| | |
|---|---|
| Tg | = 168 °C |
| $K_q$ | = 2300 psi.in.$^{0.5}$ |
| Flex Modulus | = 525 KSI |
| Flex Strength | = 15.8 KSI |
| Flex Elongation | = 3.0% |
| Gel Content | = 90% |

Illustrative Embodiment III

In Illustrative Embodiment III various polymers were prepared in a manner similar to that described in Illustrative Embodiment II.

The various materials utilized herein are described below in the Structure Legend:

STRUCTURE LEGEND

| Type | Symbol | Chemical Name |
|---|---|---|
| Phenols | BP | 4,4'-Dihydroxybiphenyl |
| | BPA | 2,2-Bis(4-hydroxyphenyl)propane |
| | BPC | 4,4'-Dihydroxybenzophenone |
| | HFBPA | 1,1,1,3,3,3-Hexafluoro-2,2-bis(4-hydroxyphenyl)propane |
| | BPFL | 9,9-Bis(4-hydroxyphenyl)fluorene |
| | BPAQ | 9,9-Bis(4-hydroxyphenyl)-10-anthrone |
| Epoxides | DGBPA | 2,2-Bis[4-(2,3-epoxypropoxy)phenyl]propane |
| | DGBPFL | 9,9-Bis[4-(2,3-epoxypropoxy)phenyl]fluorene |
| | DGBP | 4,4'-Bis(2,3-epoxypropoxy)biphenyl |
| | DGBPAQ | 9,9-Bis[4-(2,3-epoxypropoxy)phenyl]-10-anthrone |
| | DGBNDB | α,α'-bis[1-(2,3-epoxypropoxy)-2-naphthyl]-para-diisopropylbenzene |

| Type | Symbol | Chemical Name |
|---|---|---|
| Amines | ADA | Aminoadamantane |
| | ACPH | N-(4-amino-2-methylphenyl)-4-chlorophthalimide |
| | NAPH | N-aminophthalimide |
| | AAP | 4-aminoacetophenone |
| | DMA | 2,6-dimethylaniline |
| | DEA | 2,6-diethylaniline |
| | DIPA | 2,6-diisopropylaniline |
| | NMADB | N,N'-dimethyl-α,α'-bis(4-aminophenyl)-p-diisopropylbenzene |
| | NEADB | N,N'-diethyl-α,α'-bis(4-aminophenyl)-p-diisopropylbenzene |
| | ADB | α,α'-bis(4-aminophenyl)-p-diisopropylbenzene |
| | NBMDA | N,N'-di-sec-butylbis(4-aminophenyl)methane |
| | BNDB | α,α'-bis(1-hydroxy-2-naphthyl)-para-diisopropylbenzene |
| Crosslinking Agents | DEADB | α,α'-bis(3,5-diethyl-4-hydroxyphenyl)-p-diisopropylbenzene |
| | DMADB | Epon Curing Agent HPT 1062 |
| | ADB | Epon Curing Agent HPT 1061 |
| | EOCN | Epon Resin DPS-164 |
| | TGTPE | Epon Resin 1031 |
| | DDS | Diaminodiphenyl sulphone |
| | TPA | α,α'α''-tris(4-hydroxyphenyl)-1,3,5-triisopropylbenzene |

The preparation details of the polymers are described in Table 1 and the properties are described in Table 2. Referring to Table 1, the basic formulation involves an amine (A), a diepoxide (E), and a crosslinking agent (X). The amount of crosslinking agent used is based on needed stoichiometric equivalents and is designated as "%x". In some cases a fraction of the amine is substituted with a different amine or with a diphenol. These components - Y and Z - are designated along with the percent employed - %Y, %Z. Also designated in Table 1 is the amine to epoxide ratio (A/E), which would also include anything substituted for amine. In addition, the % catalyst is noted where a catalyst is used. Since catalysts are not required for the typical amine/epoxy polymer, catalysts were used in only a few of the examples. When used, the catalyst was monosodium BPA or monosodium BPFL.

The cure schedule is also shown, T/t. "T" refers to temperature of cure and "t" refers to the hours at that temperature. The various cure schemes are as follows:

T/t A = 190 °C/24 hr
B = 210 °C/24 hr
C = 110 °C/16 hr + 150 °C/24 hr
D = 130 °C/72 hr + 180 °C/2 hr
E = 150 °C/16 hr + 180 °C/4 hr
F = 150 °C/16 hr + 200 °C/6 hr

The property data for the various polymers are presented in Table 2. For most of the polymers, Tg and $K_q$ are given. For some polymers solvent resistance is shown as percent weight gain when immersed in particular materials - MEK is methylethylketone, MC is $CH_2Cl_2$ and $H_2O$ is water. The polymers were checked at room temperature (RT) after reaching equilibrium (Eq). Flex data is given for both dry and hot/wet (h/w) conditions. Gel is reported in some cases. Where the value given for gel is "O", that means that the polymer is thermoplastic and has <u>not</u> been cured to a thermoset. Gel was not measured in all cases.

TABLE 1

| | COMPONENTS | | | | | | | | | | Cat | |
|---|---|---|---|---|---|---|---|---|---|---|---|
| Run No. | A | E | X | %X | Y | %Y | Z | %Z | A/E | % | T/t |
| 1 | AAP | DGBPFL | DMADB | 10 | | | | | 1.00 | 0 | F |
| 2 | AAP | DGBPFL | | | NMADB | 10 | | | 1.00 | 0 | F |
| 3 | ACPH | DGBPA | DMADB | 10 | | | | | 1.00 | 0 | A |
| 4 | NAPH | DGBPA | DMADB | 10 | | | | | 1.00 | 0 | A |
| 5 | ADA | DGBPA | ADB | 10 | | | | | 1.00 | 0 | A |
| 6 | ADA | DGBPA | ADB | 0 | | | | | 0.96 | 0 | E |
| 7 | ADA | DGBPA | ADB | 20 | | | | | 1.00 | 0 | A |
| 8 | NBMDA | DGBPFL | ADB | 10 | DEA | 15 | | | 0.96 | 0 | F |
| 9 | NBMDA | DGBPFL | ADB | 10 | DEA | 40 | | | 0.96 | 0 | F |
| 10 | NBMDA | DGBPFL | ADB | 10 | DEA | 65 | | | 0.96 | 0 | F |
| 11 | NBMDA | DGBPFL | DDS | 10 | DEA | 65 | | | 0.96 | 0 | F |
| 12 | NBMDA | DGBPFL | DDS | 20 | DEA | 55 | | | 0.96 | 0 | F |
| 13 | NBMDA | DGBPFL | DDS | 30 | DEA | 45 | | | 0.96 | 0 | F |
| 14 | NBMDA | DGBPFL | DDS | 10 | DEA | 40 | | | 0.96 | 0 | F |
| 15 | NBMDA | DGBPFL | DDS | 20 | DEA | 30 | | | 0.96 | 0 | F |
| 16 | NBMDA | DGBPFL | DDS | 30 | DEA | 20 | | | 0.96 | 0 | F |
| 17 | DEA | DGBPFL | ADB | 10 | | | | | 1.00 | 0 | E |
| 18 | DEA | DGBPFL | DMADB | 10 | | | | | 1.00 | 0 | F |

TABLE 1 (Cont'd)

| Run No. | COMPONENTS | | | | | | | | | Cat | |
|---|---|---|---|---|---|---|---|---|---|---|---|
| | A | E | X | %X | Y | %Y | Z | %Z | A/E | % | T/t |
| 19 | DEA | DGBPFL | DMADB | 10 | | | | | 1.00 | 0 | E |
| 20 | DEA | DGBPFL | DMADB | 10 | | | | | 1.00 | 100 | E |
| 21 | DEA | DGBPFL | DMADB | 10 | | | | | 1.00 | 0 | D |
| 22 | DEA | DGBPFL | DMADB | 20 | | | | | 1.00 | 0 | F |
| 23 | DEA | DGBPFL | DMADB | 10 | | | | | 1.06 | 0 | F |
| 24 | DEA | DGBPFL | TPA | 10 | | | | | 0.96 | 100 | F |
| 25 | DEA | DGBPFL | TPA | 20 | | | | | 0.96 | 100 | F |
| 26 | DEA | DGBPFL | TPA | 5 | | | | | 1.00 | 100 | F |
| 27 | DEA | DGBPFL | TPA | 10 | | | | | 1.00 | 0 | F |
| 28 | DEA | DGBPFL | TPA | 20 | | | | | 1.00 | 100 | F |
| 29 | DEA | DGBPFL | | | NMADB | 50 | | | 0.96 | 100 | F |
| 30 | DEA | DGBPFL | | | NMADB | 75 | | | 0.96 | 100 | F |
| 31 | DEA | DGBPFL | | | BPA | 25 | | | 0.96 | 50 | F |
| 32 | DEA | DGBPFL | | | BPA | 25 | | | 0.96 | 100 | F |
| 33 | DEA | DGBPFL | | | BPA | 50 | | | 0.96 | 50 | F |
| 34 | DEA | DGBPFL | | | BPA | 50 | | | 0.96 | 100 | F |
| 35 | DEA | DGBPFL | | | BPA | 75 | | | 0.96 | 100 | F |
| 36 | DEA | DGBPFL | | | BPA | 75 | | | 0.96 | 50 | F |

TABLE 1 (Cont'd)

| | COMPONENTS | | | | | | | | | Cat | |
|---|---|---|---|---|---|---|---|---|---|---|---|
| Run No. | A | E | X | %X | Y | %Y | Z | %Z | A/E | % | T/t |
| 37 | DIA | DGBPFL | DMADB | 10 | NMADB | 15 | NBMDA | 35 | 1.04 | 0 | F |
| 38 | DMA | DGBPA | DMADB | 0 | | | | | 1.00 | 0 | C |
| 40 | DMA | DGBPA | DMADB | 10 | | | | | 1.00 | 0 | C |
| 41 | DMA | DGBPA | DMADB | 15 | | | | | 1.00 | 0 | C |
| 42 | DMA | DGBPA | DMADB | 20 | | | | | 1.00 | 0 | C |
| 43 | DMA | DGBPA | DMADB | 30 | | | | | 1.00 | 0 | C |
| 44 | DMA | DGBPA | DMADB | 40 | | | | | 1.00 | 0 | C |
| 45 | DMA | DGBPAQ | DMADB | 15 | NMADB | 10 | NBMDA | 35 | 1.04 | 0 | F |
| 46 | DMA | DGBPAQ | DMADB | 15 | NMADB | 10 | NBMDA | 25 | 1.04 | 0 | F |
| 47 | DMA | DGBPFL | ADB | 15 | | | | | 1.00 | 0 | E |
| 48 | DMA | DGBPFL | DEADB | 15 | | | | | 1.00 | 0 | E |
| 49 | DMA | DGBPFL | DMADB | 10 | NMADB | 15 | NBMDA | 35 | 1.04 | 0 | F |
| 54 | DMA | DGBPFL | DMADB | 15 | NMADB | 10 | NBMDA | 35 | 1.04 | 0 | F |
| 55 | DMA | DGBPFL | DMADB | 25 | NMADB | 0 | NBMDA | 35 | 1.04 | 0 | F |
| 61 | DMA | DGBPFL | DMADB | 15 | NMADB | 10 | NBMDA | 25 | 0.96 | 0 | F |
| 62 | DMA | DGBPFL | DMADB | 15 | NMADB | 10 | NBMDA | 25 | 1.00 | 0 | F |
| 63 | DMA | DGBPFL | DMADB | 15 | NMADB | 10 | NBMDA | 25 | 1.04 | 0 | F |
| 64 | DMA | DGBPFL | DMADB | 15 | NMADB | 10 | NBMDA | 25 | 1.06 | 0 | F |

TABLE 1 (Cont'd)

| Run No. | COMPONENTS A | E | X | %X | Y | %Y | Z | %Z | A/E | Cat % | T/t |
|---|---|---|---|---|---|---|---|---|---|---|---|
| 65 | DMA | DGBPFL | DMADB | 15 | NMADB | 10 | NBMDA | 25 | 1.08 | 0 | F |
| 66 | DMA | DGBPFL | DMADB | 15 | NMADB | 10 | NBMDA | 30 | 1.04 | 0 | F |
| 73 | DMA | DGBPFL | DMADB | 15 | | | | | 0.96 | 0 | F |
| 74 | DMA | DGBPFL | DMADB | 20 | | | | | 1.00 | 0 | E |
| 75 | DMA | DGBPFL | DMADB | 15 | | | | | 1.04 | 0 | F |
| 76 | DMA | DGBPFL | DMADB | 15 | | | | | 1.08 | 0 | F |
| 77 | DMA | DGBPFL | DMADB | 15 | | | | | 1.10 | 0 | F |
| 78 | DMA | DGBPFL | PAP | 15 | | | | | 1.00 | 100 | F |
| 79 | NMADB | DGBNDB | ADB | 0 | | | | | 1.00 | 0 | A |
| 80 | NMADB | DGBNDB | ADB | 13 | | | | | 1.00 | 0 | A |
| 81 | NMADB | DGBNDB | ADB | 20 | | | | | 1.00 | 0 | A |
| 82 | NMADB | DGBNDB | ADB | 26 | | | | | 1.00 | 0 | A |
| 83 | NMADB | DGBNDB | ADB | 36 | | | | | 1.00 | 0 | A |
| 84 | NMADB | DGBNDB | ADB | 50 | | | | | 1.00 | 0 | B |
| 85 | NMADB | DGBNDB | ADB | 75 | | | | | 1.00 | 0 | B |
| 86 | NMADB | DGBNDB | ADB | 100 | | | | | 1.00 | 0 | B |
| 87 | NMADB | DGBPFL | ADB | 0 | | | | | 1.00 | 0 | A |
| 88 | NMADB | DGBPFL | ADB | 100 | | | | | 1.00 | 0 | B |
| 89 | NMADB | DGBPFL | ADB | 50 | | | | | 1.00 | 0 | B |

TABLE 2

| Run No. | Tg °C | $K_q$ PSI.in.$^{0.5}$ | % Wt Gain (RT, Eq) MEK | MC | $H_2O$ | Flex Data (KSI) E,dry | E,h/w | Stress | Strain, % | Gel % |
|---|---|---|---|---|---|---|---|---|---|---|
| 1 | 183 | 620 | | | | 570 | 430 | 18.6 | 3.5 | |
| 2 | 171 | | | | | | | | | 80 |
| 3 | 167 | | | | | | | | | 96 |
| 4 | 132 | | | | | | | | | 90 |
| 5 | 142 | 820 | | | 1.8 | 439 | 402 | 19.5 | 9 | 96 |
| 6 | 123 | | | | | | | | | 0 |
| 7 | 151 | 740 | | | 1.8 | 431 | 395 | 19.8 | 9 | 98 |
| 8 | 143 | 1200 | | | 1.1 | 432 | 360 | 19.8 | 9 | 92 |
| 9 | 150 | 1100 | | | 1.4 | 434 | 371 | 20.2 | 7 | 92 |
| 10 | 158 | 750 | | | 1.4 | 440 | 387 | 20.8 | 9 | 93 |
| 11 | 155 | | | | | | | | | 75 |
| 12 | 161 | | | | | | | | | 90 |
| 13 | 173 | | | | | | | | | 95 |
| 14 | 150 | | | | | | | | | 76 |
| 15 | 157 | | | | | | | | | 89 |
| 16 | 163 | | | | | | | | | 92 |
| 17 | 160 | | | | | | | | | 85 |
| 18 | 158 | | | | | | | | | 85 |

TABLE 2 (cont'd)

| Run No. | Tg °C | $K_q$ $PSI.in.^{0.5}$ | % Wt Gain (RT, Eq) MEK | MC | $H_2O$ | Flex Data (KSI) E,dry | E,h/w | Stress | Strain, % | Gel % |
|---|---|---|---|---|---|---|---|---|---|---|
| 19 | 158 | | | | | | | | | 85 |
| 20 | 162 | | | | | | | | | 91 |
| 21 | 165 | 1000 | | | | 477 | | 15 | 3 | 80 |
| 22 | 164 | | | | | | | | | 93 |
| 23 | 157 | | | | | | | | | 74 |
| 24 | 158 | | | | | | | | | 95 |
| 25 | 162 | | | | | | | | | 97 |
| 26 | 156 | | | | | | | | | 88 |
| 27 | 155 | | | | | | | | | 92 |
| 28 | 160 | | | | | | | | | 94 |
| 29 | 163 | | | | | | | | | 73 |
| 30 | 161 | | | | | | | | | 0 |
| 31 | 161 | 800 | | | 1.7 | 439 | 391 | 17.1 | 5 | 51 |
| 32 | 163 | | | | | | | | | 93 |
| 33 | 166 | 900 | | | 1.9 | 427 | 387 | 12.5 | 3 | |
| 34 | 171 | | | | | | | | | 80 |
| 35 | 173 | | | | | | | | | 88 |
| 36 | 170 | 1450 | | | 2 | 419 | 377 | 9.3 | 2 | |

TABLE 2 (cont'd)

| Run No. | Tg °C | $K_q$ PSI.in.$^{0.5}$ | % Wt Gain (RT, Eq) MEK | MC | $H_2O$ | Flex Data (KSI) E,dry | E,h/w | Stress | Strain, % | Gel % |
|---|---|---|---|---|---|---|---|---|---|---|
| 37 | 160 | | | | | | | | | |
| 38 | 110 | 2200 | | | | 425 | | 16.8 | >8 | 0 |
| 40 | 125 | 2500 | 77 | 207 | | 405 | 270 | 17.1 | >8 | 86 |
| 41 | 130 | 2550 | 88 | 229 | 1.9 | 410 | 300 | 17.3 | >8 | 85 |
| 42 | 128 | 2500 | 63 | 167 | | 420 | 270 | 18.5 | >8 | 91 |
| 43 | 139 | 1600 | | | | 430 | | 18.3 | >8 | 99 |
| 44 | 143 | 1440 | | | | 435 | | 18.6 | >8 | 97 |
| 45 | 168 | | | | | | | | | |
| 46 | 178 | | | | | | | | | |
| 47 | 174 | | | | | | | | | |
| 48 | 174 | | | | | | | | | |
| 49 | 156 | | | | | | | | | |
| 54 | 161 | | | | | | | | | |
| 55 | 171 | | | | | | | | | |
| 61 | 168 | | | | | | | | | 91 |
| 62 | 170 | | | | | | | | | 98 |
| 63 | 175 | | | | | | | | | 99 |
| 64 | 164 | | | | | | | | | |

TABLE 2 (cont'd)

| Run No. | Tg °C | $K_q$ PSI.in.$^{0.5}$ | % Wt Gain (RT, Eq) MEK | MC | $H_2O$ | Flex Data (KSI) E,dry | E,h/w | Stress | Strain, % | Gel % |
|---|---|---|---|---|---|---|---|---|---|---|
| 65 | 159 | | | | | | | | | |
| 66 | 166 | | | | | | | | | |
| 73 | 174 | | | | | | | | | |
| 74 | 178 | | 22 | 157 | 1.7 | | | | | 94 |
| 75 | 188 | | | | | | | | | 92 |
| 76 | 186 | | | | | | | | | |
| 77 | 168 | | | | | | | | | 81 |
| 78 | 176 | | | | | | | | | |
| 79 | 155 | 2300 | | | 1.1 | 512 | 485 | 13.3 | 3 | |
| 80 | 163 | | 116 | 693 | | | | | | 78 |
| 81 | 163 | | 103 | 470 | | | | | | 85 |
| 82 | 168 | 2300 | 87 | 348 | 0.8 | 525 | | 15.8 | 3.0 | 90 |
| 83 | 171 | | | | | | | | | 92 |
| 84 | 177 | 1700 | | | 1 | 540 | 493 | 17.9 | 3.4 | 96 |
| 85 | 190 | 1200 | | | 1 | 544 | 493 | 17 | 3.2 | |
| 86 | 211 | 840 | | | 1.1 | 550 | 499 | 16.8 | 3.1 | |
| 87 | 161 | 1800 | | | 1.3 | 436 | 404 | 20 | 7 | |
| 88 | 246 | 445 | | | 2 | 469 | 367 | 17.8 | 5 | |
| 89 | 198 | 620 | | | 1.6 | 455 | 401 | 22.3 | 9 | |

## Claims

1. A composition comprising linear molecules having the repeating structures prior to crosslinking of the general formula:

$$-\!\!\left[-A-CH_2-\underset{OH}{\underset{|}{CH}}-CH_2-B-CH_2-\underset{OH}{\underset{|}{CH}}-CH_2-\right]\!\!-$$

where A is selected from the group consisting of:

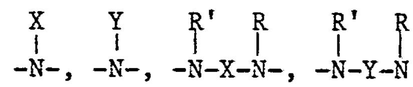

and mixtures thereof, and B is selected from the group consisting of:

-O-X-O, O-Y-O, 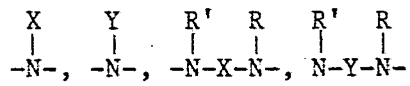

where R and R′ are selected from the group consisting of unsubstituted or inertly substituted $C_1$-$C_{20}$ aliphatic, cycloaliphatic or aromatic hydrocarbyl groups, and wherein said repeating structures are lightly crosslinked such that between 1 and 50 of said repeating structures per 100 total repeating structures are crosslinked to repeating structures of other molecules, and where:

a) "X" represents a stiff segment comprising stiff units (SU) and optional flexible units (FU), which stiff units and flexible units are interconnected;

b) "Y" represents a flexible segment comprising stiff units (SU′) and optional flexible units (FU′) which stiff units and flexible units are interconnected;

c) said stiff units, SU and SU′, are independently selected from the group consisting of substituted and non-substituted aromatic rings, cycloaliphatic rings and non-interfering heterocyclic rings;

d) said flexible units, FU and FU′, are independently selected from the group consisting of

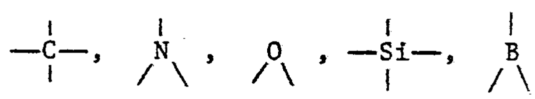 and 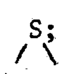

e) the number of stiff segments in the resulting polymer compositions is "a", the number of flexible segments in the resulting polymer compositions is "b", and the ratio of $\frac{a}{a+b}$ is less than or equal to one; and

f) the ratio of the number of stiff units to flexible units in said stiff segment (SU/FU) is equal to or greater than the ratio of the number of stiff units to flexible units in said flexible segment (SU′/FU′).

2. The composition of claim 1 wherein the average number of total stiff units divided by the average number of total flexible units is equal to or greater than 2 and less than about 20.

3. The composition of claim 1 or 2 wherein the ratio of

$$\frac{SU}{FU} > \frac{SU'}{FU'} + 0.5.$$

4. A composition as claimed in any one of claims 1 to 3 wherein A is selected from

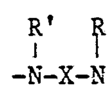 and 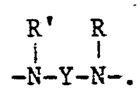

5. A composition as claimed in any one of claims 1 to 4 wherein B is selected from -O-X-O-and -O-Y-O.

6. A composition as claimed in any one of claims 1 to 5 wherein A is

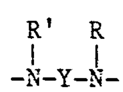

and B is -O-X-O.

7. A composition as claimed in any one of claims 1 to 6, which additionally contains an effective amount of crosslinking agent such that upon curing between 2 and 40 repeating structures from different molecules per 100 of said repeating structures are crosslinked.

8. A composition resulting from the curing of the composition of claim 7.

9. A composition as claimed in any one of claims 1 to 8 wherein between 25 and 75 mole per cent of A is replaced with a group selected from the group consisting of -O-X-O-and O-Y-O-.

10. A composition as claimed in any one of claims 1 to 9 and having a glass transition temperature below 150 °C.

11. A composition as claimed in any one of claims 1 to 10, containing N,N'-dimethyl-α,α'-bis(4-aminophenyl)-p-diisopropylbenzene as crosslinking agent.

12. A composition as claimed in any one of claims 1 to 10 containing α,α'-bis[1-(2,3-epoxypropoxy)-2-naphthyl]-para-diisopropylbenzene as crosslinking agent.

13. A composition as claimed in any one of claims 1 to 10 containing a multifunctional amine of the formula

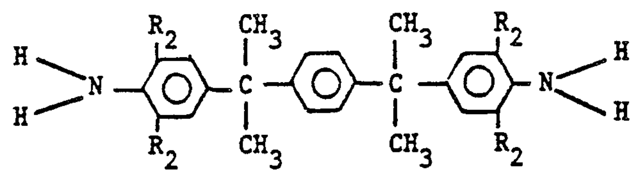

where $R_2$ is H or $CH_3$, as cross-linking agent.